# EUROPEAN PATENT APPLICATION

(11) **EP 4 279 599 A1**
(43) Date of publication of application: **22.11.2023**
(21) Application number: 22174142.4
(22) Date of filing: 18.05.2022
(51) Int. Cl.: C12N 15/88

(54) **ORDERED FORMULATION METHOD TO CONSTRUCT POLYPLEX WITH CORE-AGGREGATE STRUCTURE**

(71) Applicant: Branca Bunus Limited, A94 W1K6 Dublin (IE)
(72) Inventor: Wenxin, Wang, Dublin, 4 (IE); Yinghao, Li, Dublin, 4 (IE); Zhonglei, He, Dublin, 4 (IE); Hongyun, Tai, Dublin, 4 (IE)
(74) Representative: Tomkins & Co

(57) **Abstract**

The present invention relates to a polyplex of a nucleic acid and at least one cationic polymer and a method for producing same. Polyplexes of the invention demonstrate increased stability and high transfection efficiencies.

## Description

### Field of the Invention

The present invention relates to a method for preparing polyplexes, which may be used for transfection.

### Background to the Invention

Numerous medical diseases arise due to abnormalities in an individual's genetic code, leading to mutations that culminate in genetic disorders. Such genetic diseases account for considerable social and economic burdens globally and lead to severe lifelong negative impacts on the livelihood of suffering patients.

The ability to restore a protein's expression ("gene addition") or edit one's genetic code ("gene editing") to correct such disease-causing mutations has long been heralded as a transformative medical treatment with the potential to cure a multitude of genetic conditions. However, a substantial challenge to the broad spectrum uses of gene therapy as a first in line treatment strategy remains. The delivery of a gene to a patient's cells is not possible on its own due to physicochemical barriers preventing entry of nucleic acids into cells.

When used as transection vectors for gene delivery, conventional inactivated virus vectors introduce substantial safety concerns, such as immunogenicity and insertional mutagenesis. Moreover, they often have high production costs. As a result, treatment systems constructed with non-viral vectors are becoming more promising candidates.

Polymer vectors have been widely studied due to their easy modification and large-scale production ability. In particular, treatments offered by cationic polymer complexes (polyplexes) have the advantages of stable structure, easy mass production, easy modification, and increased stability compared to cationic liposomal DNA complexes (lipoplexes).

To achieve a good therapeutic effect, it is necessary to construct an efficient polyplex. However, the mixing method of the polymer carrier and DNA or other cargo greatly affects the performance of the polyplex. Depending on the formulation conditions, the resulting polyplex can vary in size and internal structure; it may form a spherical shape, doughnut, or other structures. As a result the transfection efficiency is often unstable.

Due to insufficient understanding of polyplex structures, polyplexes are still most commonly formed by simple direct mixing of polymer and cargo solutions.

While microfluidic and manual mixture methods are available, these often lead to instabilities in the size and structure of the polyplex and result in unreliable transfection efficiency.

Since manual mixing methods often result in unstable products, microfluidics systems for formulating polyplexes were designed. DNA and polymer solutions are mixed in a microfluidics chip at a constant speed to obtain polyplexes with relatively uniform sizes and structures. However, this method can only improve the uniformity of the size of the polyplexes and does not achieve an optimal structure. The transfection efficiency of the resulting polyplexes also still require improvement.

To improve the transfection efficiency of the polyplex, some researchers constructed polymer-lipid nanoparticles by involving a liposome shell on the polyplex. The extra lipid layer can help intracellular processes, such as cellular uptake and lysosome escape. However, the introduction of liposomes will hinder the stability of the polyplex.

Although structural differences of polyplexes will greatly affect their transfection efficiency, research on the internal structure of the polyplex is still at the early stage due to the complex structure of the polymers. Since it is impossible to observe a clear and orderly internal structure through microscopes (different from liposomes, the membrane structure can be observed), the current understanding of polyplex structure is still the chaotic structure of random entanglement of polymer and cargos.

Langer et al. pointed out that the mixing order of polymer vector and DNA will affect the efficiency of the resulting polyplex. The polyplexes displayed the highest transfection efficiency when made by slowly adding the polymers into DNA in a sodium acetate buffer.

It is clear that methods to reliably produce polyplexes having high transfection efficiency are in short supply. The present invention aims to address this issue.

### Summary of the Invention

In one aspect, the present invention provides a method for producing a polyplex of a nucleic acid and at least one cationic polymer comprising the steps of:
a) mixing a nucleic acid with a first cationic polymer in a weight ratio of from about 0.1:1 to about 10:1 respectively to form a polymer-nucleic acid core;
b) mixing the polymer-nucleic acid core with a second cationic polymer in a weight ratio of from about 1:5 to about 1:200 respectively.

The resulting polyplex may be suitable for use as a vector for transfection.

Suitably, the nucleic acid and the first cationic polymer are mixed in a weight ratio of from about 0.5:1 to about 5:1 or from 1:1 to 2.5:1 respectively.

Preferably, the nucleic acid and the first cationic polymer are mixed in a weight ratio of about 1:1 respectively.

Suitably, the polymer-nucleic acid core and the second cationic polymer are mixed in a weight ratio of from about 1:50 to about 1:150 respectively. Preferably, they are mixed in a weight ratio of from about 1:75 to about 1:100 respectively. Particularly preferred is a weight ratio of about 1:30.

The nucleic acid used may be DNA.

Alternatively the nucleic acid used may be RNA.

Where the nucleic acid used is DNA, the resulting polyplex may be suitable for delivering DNA to a cell. Where the nucleic acid used is RNA, the resulting polyplex may be suitable for delivering RNA to a cell.

The first cationic polymer may be selected from the group comprising: poly(β-amino) esters (PAE), poly(amido amine)s (PAA), poly[2-(dimethylamino)ethyl methacrylate] (PDMAEMA), polyethylenimine (PEI), or poly-L-lysine (PLL), or a combination thereof.

The second cationic polymer may be selected from the group comprising: poly(β-amino) esters (PAE), poly(amido amine)s (PAA), poly[2-(dimethylamino)ethyl methacrylate] (PDMAEMA), polyethylenimine (PEI), or poly-L-lysine (PLL), or a combination thereof.

The first and second cationic polymers may be the same.

Alternatively, the first and second cationic polymers may be different.

For example, the first cationic polymer may be a poly(amido amine)s (PAA), poly(amido amine)s (PAA), and the second cationic polymer may be a poly-L-lysine (PLL) or vice versa. Or the first cationic polymer may be a poly(amido amine)s (PAA) and the second cationic polymer may be a poly(amido amine)s (PAA) or vice versa. Other combinations are of course also suitable.

The first and second cationic polymers may have the same structural formula but have different molecular weights.

Suitably, the first cationic polymer is a poly(β-amino) ester (PAE) and the second cationic polymer is a poly(β-amino) ester (PAE) with a different molecular weight to that of the first cationic polymer.

Preferably, the second cationic polymer has a higher molecular weight than the first cationic polymer. A relatively low molecular weight, for example a molecular weight of below about 10,000 g/mol, of the first cationic polymer may provide greater flexibility to bind and condense DNA. A relatively high molecular weight of the second cationic polymer, for example a molecular weight of greater than about 20,000 g/mol, may provide increased stability and limit hydrolysis of the polymer in culture media.

The first cationic polymer and second cationic polymer may have a molecular weight ratio of from about 1:1 to about 1:200, suitably from about 1:5 to 1:20, preferably about 1:10.

The first cationic polymer may have a molecular weight of from about 800 g/mol to about 40,000 g/mol, suitably from about 1000 g/mol to about 20,000 g/mol, preferably from about 2000 g/mol to about 10,000 g/mol.

The second cationic polymer may have a molecular weight of from about 10,000 g/mol to about 100,000 g/mol, suitably from about 20,000 g/mol to about 80,000 g/mol, preferably from about 20,000 g/mol to about 60,000 g/mol.

The first cationic polymer may be a poly(β-amino) ester (PAE). Suitably, the first cationic polymer may be a PAE with a molecular weight of from about 1000 g/mol to about 10,000 g/mol, more suitably from about 2000 g/mol to about 8000 g/mol. Preferably, the first cationic polymer may be a PAE with a molecular weight of from about 3000 g/mol to about 6000 g/mol.

The second cationic polymer may be a poly(β-amino) ester (PAE). Suitably, the second cationic polymer may be a PAE with a molecular weight of from about 10,000 g/mol to about 100,000 g/mol, more suitably from about 20,000 g/mol to about 80,000 g/mol. Preferably, the second cationic polymer may be a PAE with a molecular weight from about 20,000 g/mol to about 40,000 g/mol.

Suitably, the first cationic polymer and second cationic polymer are both poly(β-amino) esters (PAE), wherein the second cationic polymer has a higher molecular weight than the first cationic polymer.

Preferably, the first cationic polymer and second cationic polymer are both poly(β-amino) esters (PAE), wherein the first cationic polymer has a molecular weight of from about 3000 g/mol to about 6000 g/mol and the second cationic polymer has a molecular weight of from about 20,000 g/mol to about 40,000 g/mol.

Even more preferably, the first cationic polymer and second cationic polymer are both poly(β-amino) esters (PAE), with 1,4-butanediol diacrylate (BDA), 5-amino-1-pentanol (S5), and entaerythritol tetraacrylate (PTTA) as the skeleton, and 1-(3-aminopropyl)-4-methylpiperazine (E7) as the endcap, wherein the first cationic polymer has a molecular weight of from about 3000 g/mol to about 6000 g/mol and the second cationic polymer has a molecular weight of from about 20,000 g/mol to about 40,000 g/mol.

Suitably, either the first cationic polymer or the second cationic polymer or both has a polydispersity index (PDI) of lower than about 3.

Step (a) may be carried out in an ionic buffer. For example, the nucleic acid, the first cationic polymer, or both may be in a solution of ionic buffer.

Similarly, step (b) may be carried out in an ionic buffer. For example, the polymer-nucleic acid core formed in step (a), the second cationic polymer, or both may be in a solution of ionic buffer.

The ionic buffer may be selected from the group comprising: calcium acetate buffer, phosphate-buffered saline (PBS), sodium chloride buffer, magnesium acetate buffer, or sodium acetate buffer. Suitably, either step (a) or step (b) or both are carried out in sodium acetate buffer.

A method of the invention may further comprise the step of:
c) mixing the polyplex formed in step (b) with a third cationic polymer in a weight ratio of from about 1:5 to about 1:200 respectively.

Suitably, the polyplex formed in step (b) and the third cationic polymer are mixed in a weight ratio of from about 1:50 to about 1:150 respectively. Preferably, they are mixed in a weight ratio of from about 1:75 to about 1:100 respectively. Particularly preferred is a weight ratio of about 1:30.

The third cationic polymer may be the same as the second cationic polymer or it may be different. The third cationic polymer may be selected from the group comprising: poly(β-amino) esters (PAE), poly(amido amine)s (PAA), poly[2-(dimethylamino)ethyl methacrylate] (PDMAEMA), polyethylenimine (PEI), or poly-L-lysine (PLL), or a combination thereof.

Suitably, the third cationic polymer may have a different molecular weight to the first cationic polymer, the second cationic polymer, or both.

A method of the invention may comprise repeating step (c) one or more times. This could be done using the third cationic polymer, or different polymers could be used in each repetition, i.e. a fourth cationic polymer, a fifth cationic polymer, etc. This allows the polyplex to be carefully assembled layer-by-layer.

Whilst not wishing to be bound by theory, it is believed that the use of a two-step method may provide a polyplex with a more ordered structure than can be achieved with traditional methods of synthesis. The use of a similar amount (by weight) of nucleic acid and first cationic polymer in step (a) leads to the formation of a smaller number of cores than in traditional methods; this may achieve a higher level of cargo entanglement on each core, which may in turn reduce the instability and unreliable transfection effects seen with previously-known polyplexes.

In another aspect, the present invention relates to a polyplex formed by the method described herein.

In one aspect, the present invention relates to a polyplex of a nucleic acid and at least one cationic polymer, comprising:
a) a core formed of a nucleic acid and a first cationic polymer; and
b) an outer layer of a second cationic polymer.

The nucleic acid and first cationic polymer may be present in the core in a weight ratio of from about 0.1:1 to about 10:1 respectively, suitably in a weight ratio of from about 0.5:1 to about 5:1 respectively.

The second cationic polymer may be present in the polyplex in a weight ratio of from about 1:30 to about 200:1 in relation to the first cationic polymer, suitably in a weight ratio of from about 1:30 to about 1:5 respectively, preferably in a weight ratio of from about 1:10 to about 1:5 respectively.

The nucleic acid of a polyplex of the invention is defined as above.

The first cationic polymer and second cationic polymer are defined as above.

A polyplex of the invention may further comprise at least one additional outer layer. For example, a polyplex may comprise one additional outer layer of a third cationic polymer, as defined above. Further additional outer layers may also be present, with a fourth cationic polymer, a fifth cationic polymer, etc. as defined above.

When used to transfect HEK293 cells, a polyplex of the invention may achieve a percentage of transfected HEK293 cells of greater than about 70% when tested using flow cytometry. The transfection efficiency for other cell lines may differ. Flow cytometry methods are described herein.

While not wishing to be bound by theory, it is believed that polyplexes of the invention may have cores which are smaller and more uniformly sized than those created by previously known methods, and this may lead to the polyplexes of the invention achieving high transfection efficiencies.

A polyplex of the invention may be stable for at least 2 hours, suitably at least 3 hours, in aqueous solution at 37 °C.

### Definitions

Given below are the condensed (by no means exhaustive) customary definitions known in the art, of which certain terms which may aid in the description of the invention.

'Polyplex' refers to a complex of cationic polymer and nucleic acid. A polyplex may be used to deliver exogenous DNA or RNA to cells through transfection.

'Cargo' refers to the portion of nucleic acid which is present in the polyplex and which can be delivered to cells by transfection. The cargo is believed to be held in the polyplex by entanglement with the cationic polymer(s).

'Core' refers to a small local aggregation formed between the nucleic acid and the first cationic polymer.

### Brief Description of the Drawings

Embodiments of the invention will be described, by way of example only, with reference to the accompanying drawings in which:

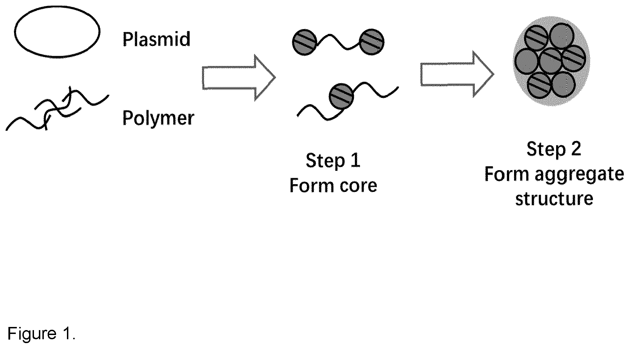

**Figure 1** is an illustration of the formation of a polyplex according to a method of the invention;
**Figure 2** is an illustration of a method according to the invention with an exemplary weight ratio;
**Figure 3** shows GPE traces for polyplexes of PAE-1 and PAE-2;
**Figure 4** shows transfection results of polyplexes of PAE-1, PAE-2 and their combination tested on HEK293 cells: (A) representative results of transfected cells with different polyplexes; (B) GFP fluorescence integrated density quantified by Image J; and (C) corresponding cell viability.
**Figure 5** shows transfection results of polyplexes of PAE-1, PAE-2 and their combination tested on HEK293 cells after the polyplexes had been incubated at 37 °C for 3 hours before transfection: (A) GFP fluorescence integrated density quantified by Image J; (B) representative results of transfected cells with different polyplexes.
**Figure 6** shows the size distribution of polyplexes of PAE-1, PAE-2 and their combination at various time points after incubation at 37 °C.

### Detailed Description of the Drawings

Two batches of poly(β-amino esters) [PAE-1 and PAE-2], with different molecular weights, were synthesised. PAE-1 and PAE-2 possessed the same structure [with 1,4-butanediol diacrylate (BDA), 5-amino-1-pentanol (S5), and entaerythritol tetraacrylate (PTTA) as the skeleton, and 1-(3-aminopropyl)-4-methylpiperazine (E7) as the endcap].

PAEs were synthesized through a facile Michael addition reaction. The A2+Bn+C2 strategy was used, in which the BDA referred to A2, S5 referred to C2, and tetrafunctional monomers PTTA referred to B4 (Bn), respectively. BDA (3.96 g), S5 (2.37 g), and PTTA (0.59 g) were dissolved in DMSO (14.70 mL). The solution was bubbled under argon for 15 mins and then the reaction occurs at 90 °C. Agilent 1260 Infinite gel permeation chromatography (GPC) and nuclear magnetic resonance (NMR) were used to monitor the reaction. The reaction was stopped by diluting the mixture to 100 mg/mL with DMSO when the Mw was approaching 10 kDa. E7 (3.14 g) was added to end-cap the acrylate terminated base polymer at room temperature for 48 h. After that, polymers were precipitated into diethyl ether three times and dried under vacuum before being stored at -20 °C.

Before use in the method of the invention, the number average molecular weight (Mn), weight average molecular weight (Mw), and polydispersity index (PDI) of PAE-1 and PAE-2 were determined by GPC equipped with a refractive index detector (RI), a viscometer detector (VS DP) and a dual angle light scattering detector (LS 15° and LS 90°). To monitor the molecular weight of polymers during the polymerization process, 20 µL of the reaction mixture was collected at different time points, diluted with 1 mL of DMF, filtered through a 0.2 µm filter and then measured by GPC. The columns (PolarGel-M, Edinburgh, UK, 7.5 mm x 300 mm, two in series) were eluted with DMF and 0.1% LiBr at a flow rate of 1 mL/min at 60 °C. Columns were calibrated with linear poly(methyl methacrylate) (PMMA) standards.

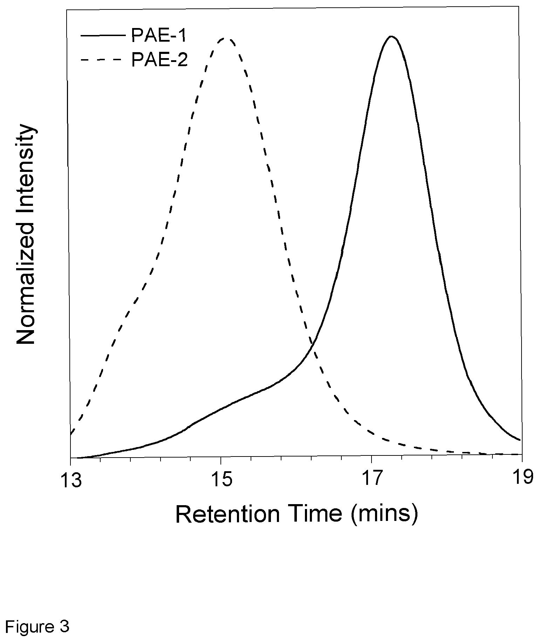

Table 1 and Figure 3 shows the GPC results of PAE-1 and PAE-2. They have the same chemical structure and monomer composition.

Table 1 - GPC results of the PAEs used in the following tests.

| | **Mn** | **Mw** | **PDI** |
|---|---|---|---|
| **PAE-1** | 3052 | 6893 | 2.2 |
| **PAE-2** | 21297 | 63688 | 3.0 |

A two-step method according to the invention was carried out to construct polyplexes with ordered structures.

The polyplexes were prepared from PAE-1, PAE-2 and gwiz-GFP plasmids in NaOAc 25 mM buffer. Eight polyplex samples were prepared, as shown in Table 2. Samples were made with PAE-1 as both the first cationic polymer and second cationic polymer, with PAE-2 as both the first cationic polymer and second cationic polymer, with PAE-1 as the first cationic polymer and PAE-2 as the second cationic polymer, and with a mixture of PAE-1 and PAE-2 as both the first cationic polymer and second cationic polymer. Polyplexes were prepared with two different weight ratios.

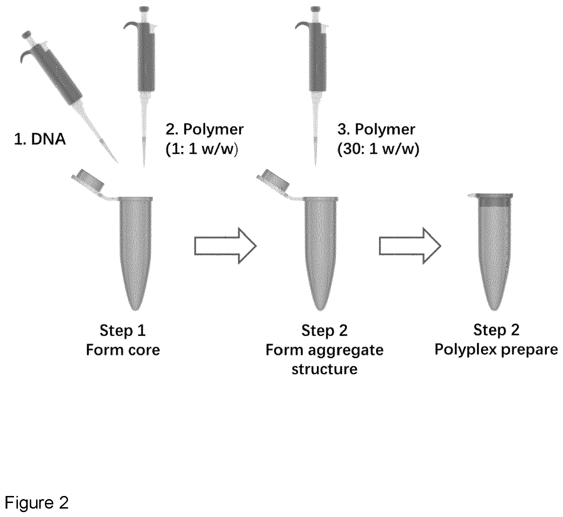

The polymers were initially dissolved in DMSO to make stock solutions (100 mg/ml), and then the stock solutions were further diluted with 25 mM sodium acetate buffer to give the desired concentration ratio for the polyplex. A solution of the DNA at 0.1 mg/ml was prepared with sodium acetate buffer. To make the polyplexes, the first cationic polymer solution was mixed with the solution of DNA in an equal volume to achieve a polymer/DNA weight ratio of 1:1 and vortexed for 10 s. After 5 min incubation at room temperature, the solution of the second cationic polymer is added to form the final polyplex, vortexed for 10 s, and allowed to stand for 10 minutes. The method is illustrated in Figure 2.

The mixture of PAE-1 and PAE-2 used in Examples 7 and 8 was prepared by directly mixing PAE-1 and PAE-2 in a DMSO stock solution at a weight ratio of 1:5 or 1:10.

**Table 2**

| **Example** | **First cationic polymer (P1)** | **Second cationic polymer (P2)** | **Weight ratio DNA: P1 : P2** |
|---|---|---|---|
| **1** | PAE-1 | PAE-2 | 1 : 1 : 10 |
| **2** | PAE-1 | PAE-2 | 1 : 1 : 5 |
| **3** | PAE-2 | PAE-2 | 1 : 1 : 10 |
| **4** | PAE-2 | PAE-2 | 1 : 1 : 5 |
| **5** | PAE-1 | PAE-1 | 1 : 1 : 10 |
| **6** | PAE-1 | PAE-1 | 1 : 1 : 5 |
| **7** | Mixture PAE-1 and PAE-2 | Mixture PAE-1 and PAE-2 | 1 : 1 : 10 |
| **8** | Mixture PAE-1 and PAE-2 | Mixture PAE-1 and PAE-2 | 1 : 1 : 5 |

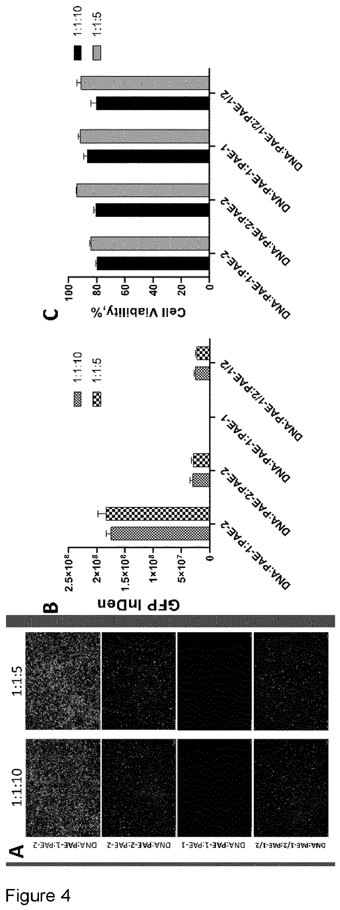

HEK293 cells were then transfected with the polyplexes of Table 2. The results are shown in Figure 4.

HEK293 cells were cultured in Dulbecco's modified Eagle Medium high glucose containing 10% fetal bovine serum and 1% Penicillin-Streptomycin. Cells were cultured at 37 °C with 5% CO₂ in a humid incubator, under standard cell culture techniques.

To perform Alamarblue assay, cell supernatants were first removed and then cells were washed with HBSS, followed by the addition of 10% Alamarblue reagent in HBSS. Living, proliferating cells maintain a reducing environment within the cytosol of the cell, converting the non-fluorescent ingredient resazurin in Alamarblue to the highly fluorescent compound resorufin. This reduction results in a color change from blue to light red and allows for the quantitative measurement of cell viability based on the increase in overall fluorescence and color of the media. The Alamarblue solution from each well was transferred to a fresh flat-bottomed 96-well plate for fluorescence measurements at 590 nm. Control cells without any treatment were used to normalize the fluorescence values and plotted as 100% viable.

As seen from Figure 4, Examples 5 and 6, which used PAE-1 as both the first cationic polymer and second cationic polymer, had no transfection efficacy at all. Without wishing to be bound by theory, this may be due to the low stability and rapid hydrolysis of small PAEs in culture media.

Examples 3 and 4, which used PAE-2 as both the first cationic polymer and second cationic polymer, presented limited efficacy. Without wishing to be bound by theory, this may be due to imperfect, randomly entangled polyplex formation caused by poor binding ability and low flexibility of large PAE molecules.

Examples 7 and 8 also presented limited efficacy. Without wishing to be bound by theory, this may be due to disordered complexation meaning that the polyplexes had too many cores, cores of varying sizes, and insufficient entanglement of the DNA.

As shown in Examples 1 and 2, when PAE-1 and PAE-2 are combined in order as described in the invention, the efficacy of transfection dramatically increased while cytotoxicity remained negligible.

Tests to show the stability of polyplexes of the invention were also carried out. A polyplex of the invention was prepared, according to the method described above, with gwiz-GFP plasmids, PAE-1 as the first cationic polymer, and PAE-2 as the second cationic polymer. The DNA:PAE-1:PAE-2 ratio was 1:2:13 (i.e. achieving an overall DNA:polymer ratio of 1:15), as shown in Example 10 of Table 3.

As comparative examples, polyplexes with PAE-1 (Example 9) and PAE-2 (Example 11) and gwiz-GFP plasmids were prepared according to a conventional one-step method, with direct mixing in a 1:15 ratio of DNA:polymer. The polymers were initially dissolved in DMSO to make stock solutions (100 mg/ml), and then the stock solutions were further diluted with 25 mM sodium acetate buffer to achieve the desired weight ratio. DNA was diluted to 0.1 mg/ml with sodium acetate buffer. The polymer solutions were added into the DNA solution, vortexed for 10 s, and allowed to stand for 15 min.

**Table 3**

| **Example** | **Method of preparation** | **First cationic polymer (P1)** | **Second cationic polymer (P2)** | **Weight ratio DNA: P1 : P2** |
|---|---|---|---|---|
| **9** | One-step | PAE-1 | n/a | 1:15 |
| **10** | Two-step | PAE-1 | PAE-2 | 1:2:13 |
| **11** | One-step | PAE-2 | n/a | 1:15 |

The polyplexes of Examples 9-11 were then incubated at 37 °C for 2 hours. At various time points, the polyplexes were used in the transfection of HEK293 cells by the method described above.

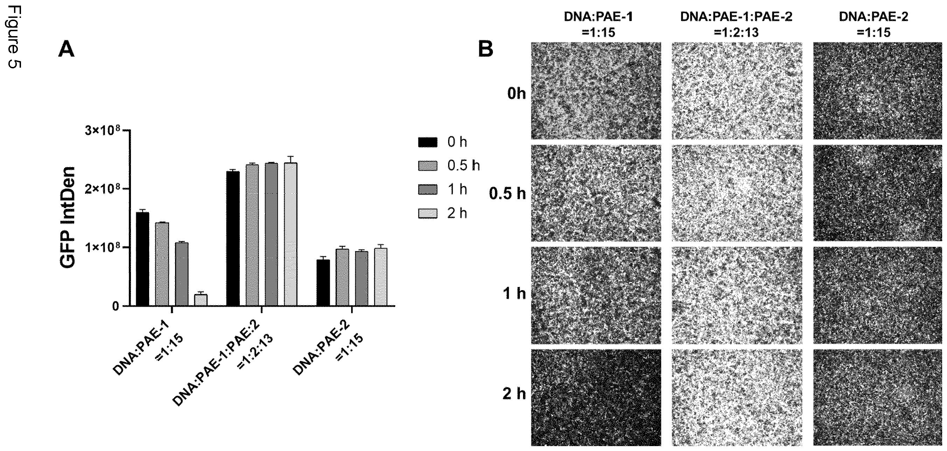

The results are shown in Figure 5. It can be seen that the inventive polyplex of Example 10 exhibited high transfection efficiency, even after 2 hours of incubation at 37 °C. At all time points observed, the polyplex of Example 10 showed significantly higher transfection efficiency than both Examples 9 and 11, which were prepared according to a conventional direct mixing method.

Furthermore, the polyplex of Example 10 showed no decrease whatsoever in transfection efficiency across the time points tested. In comparison, Example 9 showed a significant decrease in transfection efficiency as the incubation time increased.

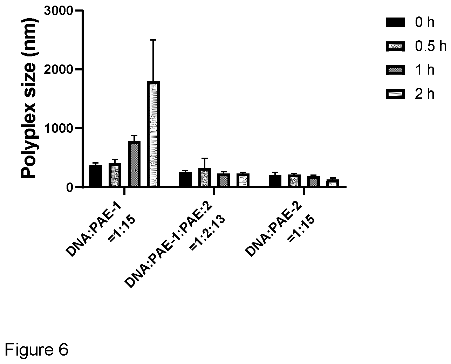

The polyplex sizes of Examples 9-11 were also assessed after incubation at 37 °C, at various time points up to 2 hours. The results are shown in Figure 6.

For the measurement of the size and zeta potential of the polyplexes, polyplexes 9-11 were prepared as described above. After that, the sizes and zeta potentials of polyplexes were measured with a Malvern Panalytical Zetasizer (ZTS1240). All the measurements were repeated three times.

The polyplexes of Examples 10 and 11 both showed high stability even after incubation. The rapid increase in polyplex size of Example 9 suggests that the polyplex aggregates during incubation, which is undesirable. Since Example 11 showed significantly lower transfection efficiency than Example 10 (as discussed above), Example 10 is the only polyplex tested which shows both high transfection efficiency and high stability after incubation.

Without wishing to be bound by theory, it is believed that when the first cationic polymer and the nucleic acid are combined, the DNA bends and entangles on the polymer to form multiple aggregate cores under electrostatic interaction. This process is carried out in ionic buffer, and the DNA finish the first stage of compression.

The introduction in step (a) of the first cationic polymer in an approximately equivalent weight to the nucleic acid is believed to limit the number of cores formed, where the polymer is the limiting reagent, compared to its use in large excess in previously reported methods. This is believed to encourage thorough entanglement of the nucleic acid on each core. Furthermore, it ensures that polyplexes made by the method of the invention have a consistent number of cores; this may eliminate the problems of unreliable/irreproducible transfection effects observed with previous methods.

When the second cationic polymer is added (in excess), it is believed to encourage the cores to further aggregate and intertwine with each other, finally forming the aggregation structure. Excess polymers entangle with each other, covering the surface of the aggregates, forming a positively charged polyplex.

The words "comprises/comprising" and the words "having/including" when used herein with reference to the present invention are used to specify the presence of stated features, integers, steps or components but do not preclude the presence or addition of one or more other features, integers, steps, components or groups thereof.

It is appreciated that certain features of the invention, which are, for clarity, described in the context of separate embodiments, may also be provided in combination in a single embodiment. Conversely, various features of the invention which are, for brevity, described in the context of a single embodiment, may also be provided separately or in any suitable sub-combination.

## Claims

1. A method for producing a polyplex of a nucleic acid and at least one cationic polymer comprising the steps of:
mixing a nucleic acid with a first cationic polymer in a weight ratio of from about 0.1:1 to about 10:1 respectively to form a polymer-nucleic acid core; and
mixing the polymer-nucleic acid core with a second cationic polymer in a weight ratio of from about 1:5 to about 1:200 respectively.

2. A method as claimed in claim 1 wherein the nucleic acid and the first cationic polymer are mixed in a weight ratio of about 1:1 respectively.

3. A method as claimed in claim 1 or claim 2 wherein the nucleic acid is DNA, or wherein the nucleic acid is RNA.

4. A method as claimed in any preceding claim wherein the first cationic polymer is selected from the group comprising: poly(β-amino) esters (PAE), poly(amido amine)s (PAA), poly[2-(dimethylamino)ethyl methacrylate] (PDMAEMA), polyethylenimine (PEI), or poly-L-lysine (PLL), or a combination thereof.

5. A method as claimed in any preceding claim wherein the second cationic polymer is selected from the group comprising: poly(β-amino) esters (PAE), poly(amido amine)s (PAA), poly[2-(dimethylamino)ethyl methacrylate] (PDMAEMA), polyethylenimine (PEI), or poly-L-lysine (PLL), or a combination thereof.

6. A method as claimed in any preceding claim wherein the first and second cationic polymers are different.

7. A method as claimed in any preceding claim wherein the first and second cationic polymers have the same structural formula but have different molecular weights.

8. A method as claimed in any preceding claim wherein the first cationic polymer is a poly(β-amino) ester (PAE) and the second cationic polymer is a poly(β-amino) ester (PAE) with a different molecular weight to that of the first cationic polymer.

9. A method as claimed in any preceding claim wherein the second cationic polymer has a higher molecular weight than the first cationic polymer.

10. A method as claimed in any preceding claim wherein the first cationic polymer and second cationic polymer may have a molecular weight ratio of from about 1:1 to about 1:200.

11. A method as claimed in any preceding claim wherein the first cationic polymer may have a molecular weight of from about 800 g/mol to about 40,000 g/mol.

12. A method as claimed in any preceding claim wherein the second cationic polymer may have a molecular weight of from about 10,000 g/mol to about 100,000 g/mol.

13. A method as claimed in any preceding claim wherein the first cationic polymer is a poly(β-amino) ester (PAE) and/or wherein the second cationic polymer is a poly(β-amino) ester (PAE).

14. A method as claimed in any preceding claim further comprising the step of:
c) mixing the polyplex formed in step (b) with a third cationic polymer in a weight ratio of from about 1:30 to about 200:1 respectively, optionally further comprising repeating step (c) one or more times.

15. A a polyplex of a nucleic acid and at least one cationic polymer, comprising:
c) a core formed of a nucleic acid and a first cationic polymer; and
an outer layer of a second cationic polymer.
